# EUROPÄISCHE PATENTSCHRIFT

(11) **EP 2 851 419 B1**
(45) Veröffentlichungstag und Bekanntmachung des Hinweises auf die Patenterteilung: **13.07.2016**
(21) Anmeldenummer: 14185121.2
(22) Anmeldetag: 17.09.2014
(51) Int. Cl.: C12M 1/107, B01F 7/00, B01F 7/18, B01F 15/00, C12M 1/06

(54) **Rührwerk für einen Fermenter und Fermenter**
Agitator for a fermenter and fermenter
Agitateur pour un dispositif de fermentation et dispositif de fermentation

(30) Priorität: 19.09.2013 DE 202013104292 U
(43) Veröffentlichungstag der Anmeldung: 25.03.2015
(73) Patentinhaber: Agraferm Technologies AG, 85276 Pfaffenhofen/Ilm (DE)
(72) Erfinder: Frankenberg, Frank, B - 4700 Eupen (BE); Harders, Dirk, D - 24107 Kiel (DE)
(74) Vertreter: Patronus IP Patent- und Rechtsanwälte

(56) Entgegenhaltungen:
- EP-B1- 2 064 308
- DE-A1-102007 034 463
- DE-U1-202004 004 101
- US-A- 4 013 274
- US-A1- 2006 215 488

## Beschreibung

Die vorliegende Erfindung betrifft ein Rührwerk für einen Fermenter und einen Fermenter mit einem solchen Rührwerk.

US 2006/025 488 A1 offenbart Halterungen für einen rotierenden Schaft und bezieht sich vor allem auf Stützlager um Rührwellen zu unterstützen.

US 5.568.985 betrifft einen Mixerapparat, insbesondere mit einem langen Rührstab mit einem Stützlager am nicht-angetriebenen Ende des Rührstabs.

Aus der DE 201 11 480 U1 geht eine Vorrichtung zum Gären organischer Substanzen hervor, welche eine motorisch antreibbare Rühreinrichtung entlang einer vertikal ausgerichteten Längsachse aufweist. Diese Rühreinrichtung weist weiterhin Rührmittel auf, die um eine Wesenlich vertikal ausgerichtete Rührachse drehbar sind. An dem unteren Ende der Rührachse ist mittels eines zweiten Achsflansches lösbar ein Kugelzapfen festgelegt. Dieser Kugelzapfen kann alternativ auch mittelbar oder unmittelbar mit der Rührachse verschweißt sein. Durch diese Art der Anbringung kann die Rühreinrichtung aus ihrer normalen vertikalen Ausrichtung verschwenkt werden.

Aus der DE 20 2004 004 101 U1 geht ein Fermenter für eine Biogasanlage mit einer Rühreinrichtung hervor, die eine vertikal stehende Rührwelle aufweist. Die Rührwelle ist an ihrem unteren Ende als Hohlwelle ausgebildet, so dass sie auf ein ortsfest im Fermenter angeordnetes Führungsrohr aufsteckbar ist. Am unteren Ende der Rührwelle ist ein nach unten sich aufweitender Einführtrichter angeordnet. An der Rührwelle sind schwenkbar ausgebildete Rührpaddel vorgesehen, so dass die Rührwelle einfach vertikal für Wartungs- oder Reparaturarbeiten entnommen werden kann.

Aus der EP 2 064 308 B1 geht ein Rührwerk für einen Fermenter hervor, das eine etwa vertikal angeordnete Rührwelle aufweist, an welcher Rührpaddel angeordnet sind. Ein Antriebsmechanismus zum Drehen der Rührwelle ist im oberen Endbereich der Rührwelle angeordnet. Ein Zentrierlager zum Zentrieren des unteren Endes der Rührwelle ist separat von der Rührwelle ausgebildet und am Boden eines Fermenters befestigbar. Dieses Zentrierlager weist einen Einführtrichter und einen Zentrierabschnitt auf, wobei der Zentrierabschnitt unterhalb des Einführtrichters angeordnet ist. Am unteren Endbereich der Rührwelle ist ein Wellenstummel ausgebildet, der drehbar gegenüber der übrigen Rührwelle mittels eines Lagers gelagert ist. Der Wellenstummel weist ein Koppelelement auf, das mit etwas Spiel formschlüssig im Zentrierabschnitt des Zentrierlagers eingreift.

Das Dokument US 4,013,274 beschreibt einen Apparat zum Führen und Einsetzen eines Rührschaftes für Labor- oder Industrieglasapparate, welcher aus Lagern und Gegenlagern besteht. Dies ermöglich es, den Böschungswinkel der Rührschaftachse in dem Apparat zu verändern.

In US 2006/0215488 A1 wird eine Stützlagerzusammensetzung beschrieben, welche leicht zu reinigen und auszutauschen ist ohne andere Teile eines Rührwerks aufteilen zu müssen.

In der DE 10 2007 034 463 A1 wird ein Gleitlager, insbesondere ein Rührwerkbodenlager für einen Reaktor beschrieben. Das Lager hat einen hülsenförmigen Lageraußenteil und einen zapfenartigen Lagerinnenteil. In einem ringförmigen Lagerspalt zwi schen diesen beiden Teilen kann ein als Schmiermittel wirkendes Fluid aufgenommen werden.

Das Rührwerk der vorliegenden Erfindung hat sich vor allem für große Fermenter mit hoch-viskosem Fermenterinhalt bewährt. Diese Rührwerke können mit geringer Drehgeschwindigkeit von zum Beispiel bis zu 20 U/min betrieben werden, so dass der Fermenterinhalt laminar umgewälzt wird.

Der Erfindung liegt die Aufgabe zugrunde, ein Rührwerk für einen Fermenter zu schaffen, das einfach in einem beladenen Fermenter ausgetauscht werden kann und eine lange Lebensdauer besitzt.

Die Aufgabe wird durch die in den unabhängigen Ansprüchen definierten Rührwerke gelöst. Vorteilhafte Ausgestaltungen sind in den jeweiligen Unteransprüchen angegeben.

Gemäß einem Aspekt der vorliegenden Erfindung umfasst ein Rührwerk für einen Fermenter nach Anspruch 1 zusätzlich:
- eine etwa vertikal angeordnete Rührwelle, an welcher zumindest ein Paddel angeordnet ist,
- einen Antriebsmechanismus zum Drehen der Rührwelle, wobei der Antriebsmechanismus am oberen Endbereich der Rührwelle angreift,
- ein Zentrierlager, das einen Zentrierabschnitt und einen Findungstrichter aufweist,
- einen Lagereinsatz, der am unteren Ende der Rührwelle angeordnet ist und einen mit dem Zentrierlager verbundenen bzw. koppelbaren Wellenstummel drehbar gegenüber der Rührwelle lagert. Das Rührwerk zeichnet sich dadurch aus, dass der Lagereinsatz lösbar mit der Rührwelle verbunden ist.

Der Lagereinsatz eines solchen Rührwerks unterliegt erheblichen mechanischen Beanspruchungen. Der Lagereinsatz kann daher verschleißen. Dies ist insbesondere der Fall, wenn das Rührwerk in einem Fermenter mit sehr zähflüssigem Fermenterinhalt (Substrat) verwendet wird. Bei einem solchen Betrieb ist es oftmals notwendig, den Lagereinatz regelmäßig auszutauschen. Die lösbare Verbindung zwischen dem Lagereinsatz und der Rührwelle kann beispielsweise durch zwei kompatible Flansche ausgebildet sein, wobei ein Flansch an der Rührwelle und der andere Flansch am Lagereinsatz angeordnet ist. Die Flansche sind derart mit Löchern versehen, dass sie mit Schrauben miteinander verschraubt werden können. Die Schraubverbindung stellt somit die lösbare Verbindung dar. Hierdurch ist ein einfaches und schnelles Wechseln der Lagereinsätze vor Ort möglich, ohne dass es notwendig ist, dass eine Rührwelle in eine Werkstatt transportiert wird, in welcher der Lagereinsatz beispielsweise mit aufwändigen Schweißarbeiten ausgetauscht wird. Schweißverbindungen sind keine lösbaren Verbindungen im Sinne der vorliegenden Erfindung. Lösbare Verbindungen sind Verbindungen, die keine materialtrennende Bearbeitung erfordern, wie Schneiden, Schneidbrennen, spanende Abtragung, Sägen oder Feilen.

Dieses Rührwerk kann mit einem am Boden eines Fermenters angeordneten Zentrierlager, bestehend aus einem Einführtrichter und einem unterhalb des Einführtrichters angeordneten Zentrierabschnittes, ausgebildet sein, wie es beispielsweise aus der EP 2 064 308 B1 bekannt ist. Dieses Rührwerk kann jedoch auch gemäß einem unten erläuterten zweiten Aspekt der vorliegenden Erfindung ausgebildet sein.

Gemäß der vorliegenden Erfindung umfasst ein Rührwerk für einen Fermenter
- eine etwa vertikal angeordnete Rührwelle, an welcher zumindest ein Paddel angeordnet ist,
- einen Antriebsmechanismus zum Drehen der Rührwelle, wobei der Antriebsmechanismus am oberen Endbereich der Rührwelle angreift,
- einen Findungstrichter, der am unteren Ende der Rührwelle angeordnet ist, wobei der Findungstrichter einen Zentrierabschnitt und einen nach unten sich aufweitenden Trichterabschnitt aufweist,
- einen an einem Boden eines Fermenters befestigbaren Zentrierstummel zum formschlüssigen Eingreifen in den Zentrierabschnitt,
- einen Lagereinsatz, der am unteren Ende der Rührwelle angeordnet ist und einen mit dem Zentrierlager verbundenen Wellenstummel drehbar gegenüber der Rührwelle lagert. Dieses Rührwerk zeichnet sich dadurch aus, dass der Findungstrichter und/oder der Zentrierabschnitt zumindest eine Öffnung aufweisen, so dass beim Eintauchen des Findungstrichters in ein, in einem Fermenter enthaltenes Substrat, dieses durch den Findungstrichter hindurch strömen kann.

Durch das Vorsehen der zumindest einen Öffnung wird mit der durch den sich nach unten hin öffnenden Findungstrichter erzeugte Strömungswiderstand in dem im Fermenter enthaltenen Substrat erheblich herabgesetzt. Durch den vom Findungstrichter verursachten Strömungswiderstand wird das Eintauchen des Rührwerkes in das Substrat gebremst, da das Substrat jedoch durch den Findungstrichter hindurch strömen kann, senkt sich das Rührwerk aufgrund seines Gewichtes in das Substrat ab, ohne dass es mit seinem unteren Ende seitlich ausbricht. Das Rührwerk kann somit einfach und zuverlässig abgesenkt werden, so dass mit dem Findungstrichter der im Fermenter bereits angeordnete Zentrierstummel sicher erfasst wird.

Die zumindest eine Öffnung im Zentrierabschnitt bzw. im Findungstrichter erlaubt, den Findungstrichter groß auszubilden. Ein kreisförmiger Findungstrichter kann mit einem Durchmesser von 30 bis 80 cm bzw. mit einer Fläche von ca. 2500 cm² bis ca. 5000 cm² ausgebildet sein. Ein quadratisch pyramidenförmiger Findungstrichter kann mit einer Kantenlänge von ca. 30 bis ca. 80 cm ausgebildet sein.

Vorzugsweise sind mehrere Öffnungen im Zentrierabschnitt und/oder im Findungstrichter ausgebildet und insbesondere sind mehrere Öffnungen sowohl im Zentrierabschnitt als auch im Findungstrichter vorgesehen.

Bei einem Rührwerk, wie es in der EP 2 064 308 B1 beschrieben ist, bei dem ein nach oben hin sich aufweitender Einführtrichter vorgesehen ist, besteht das Problem, dass sedimentierende Steine von dem Trichter aufgefangen werden und einen dort formschlüssig eingreifenden Wellenstummel zermahlen. In Biogasanlagen ist das Substrat strukturviskos, d.h., dass bei Scherbeanspruchung die Viskosität sinkt und suspendierte Steine sedimentieren. Speziell an der Rührwerkswelle wird die Fermenterflüssigkeit durch Scherung verdünnt, so dass hier Steine nach unten in den Trichter sinken. Bei dem sich nach unten hin öffnenden Findungstrichter gemäß dem zweiten Aspekt der vorliegenden Erfindung werden sinkende Steine seitlich abgelenkt und sammeln sich nicht im Bereich des Zentrierstummels an. Der Bereich, an dem der Zentrierstummel mit dem Zentrierabschnitt koppelt, wird somit frei von herabsinkenden Steinen gehalten. Dies erhöht erheblich die Lebensdauer des Rührwerkes. Die Öffnungen im Zentrierabschnitt bzw. im Findungstrichter machen es möglich, ein Rührwerk mit einem solchen Findungstrichter zügig und geradlinig in das zähflüssige Substrat einzutauchen.

Der Zentrierabschnitt weist vorzugsweise in der Draufsicht die Form eines Polyeders, insbesondere eines Quadrates, auf. Der Zentrierstummel weist vorzugsweise eine zum Zentrierabschnitt komplementäre Form auf, so dass der Zentrierabschnitt und der Zentrierstummel drehfest miteinander koppelbar sind.

Der Lagereinsatz weist vorzugsweise zumindest ein Kugel-, Wälz-, Rollen-, Pendelrollen- und/oder Nadellager auf. Das Lager ist zwischen dem Wellenstummel und einem Lagersatzgehäuse angeordnet, so dass der Wellenstummel drehbar innerhalb des Lagersatzgehäuses gelagert ist. Es können insbesondere zwei Lager zwischen dem Wellenstummel und dem Lagersatzgehäuse vorgesehen sein, die voneinander beabstandet sind. Unterhalb eines jeden Lagers ist vorzugsweise ein Dichtelement vorgesehen, das verhindert, dass Substrat in das Lager eindringen kann. Oberhalb des Lagers können Hohlräume ausgebildet sein, die als Fettreservoir dienen.

Der Findungstrichter ist vorzugsweise lösbar mit dem Wellenstummel verbunden. Dies ist insbesondere zweckmäßig, wenn der Lagereinsatz lösbar mit der Rührwelle verbunden ist, so dass der Lagereinsatz einfach vor Ort ausgetauscht werden kann.

An der Rührwelle kann zunächst ein Rührpaddel angeordnet sein, das mit unterschiedlicher Neigung durch Drehen um die horizontale Längsachse der Rührpaddel gegenüber der Vertikalen angeordnet werden kann. Dadurch ist es möglich bei laminarer Umwälzung eine Schichtenbildung in dem Fermenter einzustellen.

Zum Erzielen einer laminaren Umwälzung werden die Rührpaddel vorzugsweise langsam bewegt. Eine solche langsame Bewegung enspricht einer Drehgeschwindigkeit des Rührwerks bis max. 20 U/min.

Mehrere Rührpaddel können an frei wählbaren Positionen der Rührwelle befestigt werden, so dass einzelne Schichten des Substrates individuell mit dem Rührpaddel beaufschlagbar sind.

Ein Fermenter gemäß der vorliegenden Erfindung umfasst
- ein Gehäuse mit zumindest einer Bodenplatte und einer oder mehreren, die Bodenplatte umschließenden Seitenwandungen,
- ein Rührwerk, das nach einem der oben erläuterten Aspekte der vorliegenden Erfindung ausgebildet ist,
- eine Eintragseinrichtung zum Zuführen von Inputstoffen, und
- eine Entnahmeöffnung.

Die Verwendung der erfindungsgemäßen Rührwerke in einem solchen Fermenter, insbesondere einem Fermenter zum Fermentieren von Biomasse, und insbesondere einen Fermenter zum Erzeugen von Biogas, erlauben einen lang andauernden Betrieb, ohne dass an den Rührwerken Wartungsarbeiten anfallen. Hierdurch werden die Ausfallzeiten des Fermenters gering gehalten.

Geeignete Inputstoffe für die Trockenfermentation sind im Wesentlichen alle verwertbaren Biomassen mit einem Trockenstoff-Gehalt von zumindest 25 %. Geeignete Inputstoffe sind z. B. Silomais, Getreide-GPS, Grassilage, Zuckerrübensilage, Futterrübensilage und Getreide (Roggen, Triticale, Gerste, Weizen).

Vorzugsweise weist der Fermenter zumindest zwei Rührwerke auf. Beim Vorsehen von zwei Rührwerken ist es möglich, beim Ausfall eines Rührwerkes den Betrieb alleine mit einem einzigen Rührwerk fortzusetzen, wobei während des Betriebes mit einem Rührwerk das andere Rührwerk entnommen, gewartet und wieder eingesetzt werden kann. Hierdurch ist ein kontinuierlicher Betrieb des Fermenters sichergestellt. Dies gilt insbesondere, wenn der Lagereinsatz einfach ausgetauscht und das zu überholende Rührwerk vor Ort gewartet werden kann.

Vorzugsweise ist die Seitenwandung des Fermenters in der Draufsicht kreisförmig ausgebildet. Hierdurch ist es möglich, mit einem oder wenigen Rührwerken den vollständigen Fermenterinhalt laminar umzuwälzen.

Ein weiterer Aspekt der vorliegenden Erfindung betrifft ein Verfahren zum Erzeugen von Biogas mit einem erfindungsgemäßen Fermenter, wobei an der Eintragseinrichtung Inputstoffe zugeführt werden, im Gehäuse unter laminarer Umwälzung fermentiert und an der Entnahmeöffnung abgezogen werden.

Der erfindungsgemäße Fermenter umfasst
ein Gehäuse mit zumindest einer Bodenplatte und einer oder mehreren die Bodenplatte umschließenden Seitenwandungen,
ein Rührwerk zum Rühren des im Fermenter befindlichen Substrats,
eine Eintragseinrichtung zum Zuführen von Inputstoffen, und
eine Entnahmeöffnung, wobei die Eintragseinrichtung zum Zuführen der Inputstoffe am oberen Bereich des Fermenters und die Entnahmeöffnung am unteren Bereich des Fermenters angeordnet sind.

Der erfindungsgemäße Fermenter wird somit von oben nach unten durchströmt, was einen kontinuierlichen Betrieb ermöglicht, wobei sich im Fermenter drei Zonen mit unterschiedlich dichtem Material bilden können. Die oberste Zone ist die Verflüssigungszone, in welcher das eingebrachte Substrat verflüssigt wird. Die mittlere Zone ist die Methanisierungszone, in welcher das bereits verflüssigte und etwas verdichtete Material den größten Anteil an Methan freisetzt. In der untersten Zone, der Entnahmezone, befindet sich das bereits vollständig bzw. fast vollständig abgebaute Substrat, das die größte Dichte aufweist. Durch die Durchströmung von oben nach unten wird eine hydraulische Entkopplung der Abbauschritte erzielt, wodurch die Biogasausbeute optimiert wird.

Die einstellbare Neigung der Paddel erlaubt eine präzise Einstellung der Schichten, wobei bei unterschiedlichen Substraten unterschiedliche Neigungen zweckmäßig sind.

Bei der bevorzugten Ausführungsform des erfindungsgemäßen Fermenters ist das Rührwerk mit einer vertikal angeordneten Rührwelle ausgebildet. Hierdurch werden mit der Rührwelle verbundene Paddel jeweils in einer horizontalen Ebene bewegt und das im Fermenter befindliche Substrat wird in Horizontalebenen gerührt. Dies fördert die Einstellung der oben beschriebenen geschichteten Abbauzonen.

Das erfindungsgemäße Verfahren zum Betreiben eines Fermenters, insbesondere eines Fermenters für die Trockenfermentation für Biogasanlagen, zeichnet sich dadurch aus, dass am oberen Bereich des Fermenters Inputstoffe für die Trockenfermentation zugeführt werden, und dass am unteren Bereich des Fermenters das vergorene Substrat abgezogen wird.

Durch die Durchströmung von oben nach unten wird die oben erwähnte Zonenbildung ermöglicht.

Vorzugsweise wird das Substrat des Fermenters radial vermischt, was die Zonenbildung fördert. Hierzu ist es auch förderlich, dass das Rühren langsam, beispielsweise mit einer Drehgeschwindigkeit der Rührwelle(n) im Bereich von 0 bis 20 U/min bzw. bis maximal 60 U/min durchgeführt wird. Insbesondere mit Drehgeschwindigkeiten von < 10 U/min, < 20 U/min oder < 30 U/min.

Die Erfindung wird nachfolgend beispielhaft näher anhand der Zeichnungen erläutert. In den Zeichnungen zeigen schematisch:
- Figur 1: einen Ausschnitt eines erfindungsgemäßen Fermenters in einer Schnittdarstellung,
- Figur 2: einen Schnitt durch den unteren Endbereich eines Rührwerks, das auf einem Zentrierstummel lagert,

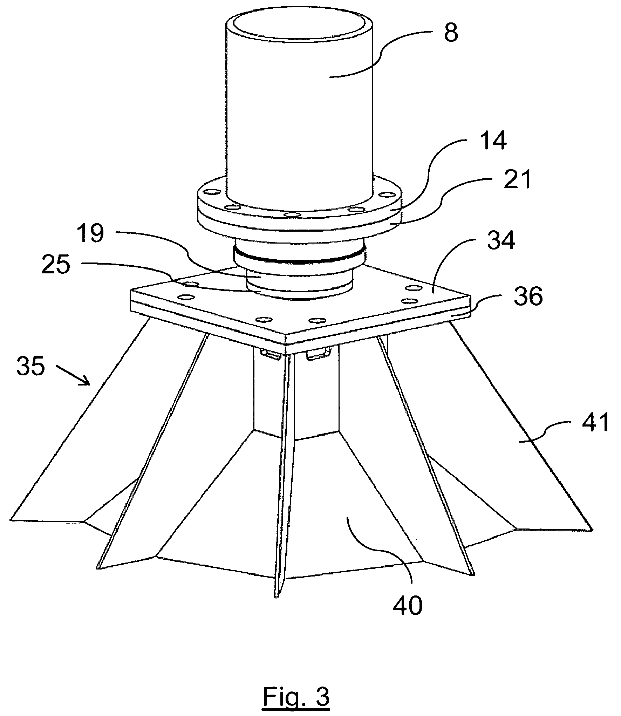

- Figur 3: den unteren Endbereich einer erfindungsgemäßen Rührwelle mit Findungstrichter in perspektivischer Ansicht,

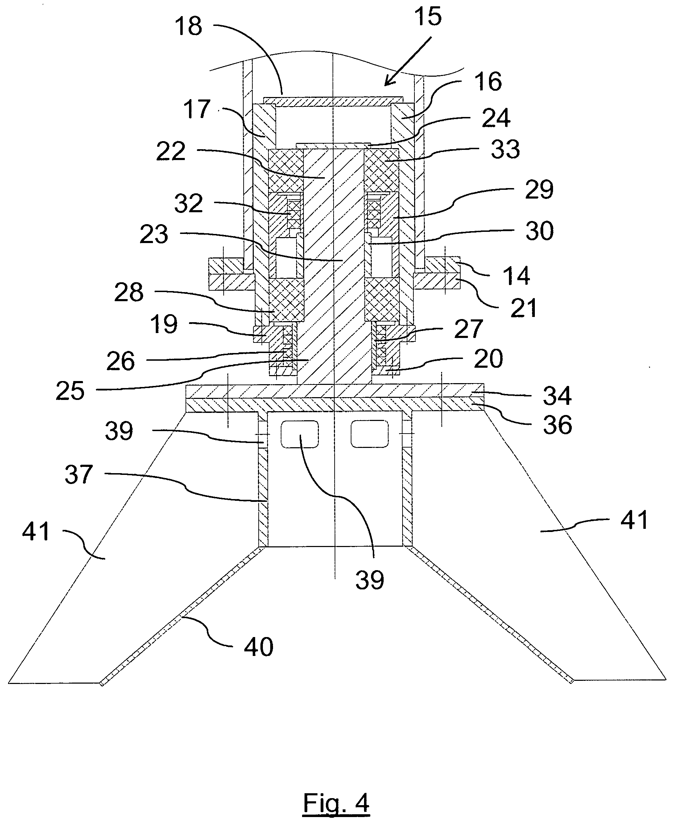

- Figur 4: den unteren Endbereich der Rührwelle aus Figur 3 in einer Schnittansicht,
- Figur 5: den Findungstrichter in einer Ansicht von oben,
- Figur 6: die Anordnung aus Figur 2 in einer isometrischen Schnittansicht, und
- Figur 7: den Zentrierstummel in einer Draufsicht.

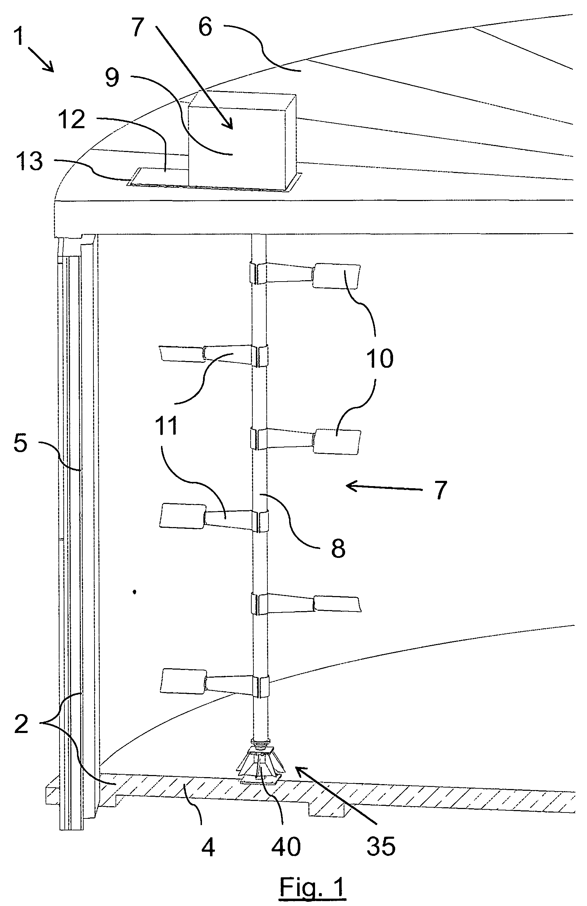

Ein Fermenter 1 weist einen Fermenterkörper 2 zur Aufnahme eines Substrates auf (Figur 1).

Der Fermenterkörper 2 ist aus einer in der Draufsicht kreisförmigen Bodenplatte 4, einer die Bodenplatte 4 umschließenden Seitenwandung 5 und einer Decke 6 ausgebildet. Die Seitenwandung 5 und die Decke 6 sind aus Fertig-Betonteilen zusammengesetzt.

Der Fermenter 1 weist zumindest ein Rührwerk 7 auf. Das Rührwerk 7 umfasst eine etwa vertikal angeordnete Rührwelle 8, einen Antriebsmechanismus 9, der am oberen Endbereich der Rührwelle 8 angreift, mehrere Rührpaddel 10, die jeweils mittels einer Paddelstange 11 an der Rührwelle 8 befestigt sind.

Der Antriebsmechanismus 9 ist aus einem Elektromotor und einem Antriebsgetriebe ausgebildet und ist oberhalb der Decke 6 angeordnet. Der Antriebsmechanismus 9 ist an einer Deckelplatte 12 befestigt, mit der eine Öffnung 13 der Decke 6 abgedeckt ist. Die Durchführung der Rührwelle 8 erfolgt mit einem gasdichten Lager. Das Substrat kommt im Normalbetrieb nicht mit der Decke 6 und der Deckelplatte 12 in Kontakt.

Die Rührwelle 8 ist aus einem Stahlrohr ausgebildet, das sich von der Decke 6 bis knapp über die Oberfläche der Bodenplatte 4 erstreckt. Die Paddelstangen 11 sind an die Rührwelle 8 geklemmt. Diese Klemmung kann an einer an sich beliebigen Stelle der Rührwelle 8 erfolgen. Deshalb ist es möglich, die Anzahl und die Anordnung der Rührpaddel 10 zu variieren. Die Rührpaddel müssen nicht, wie es in Figur 1 gezeigt ist, in einer Ebene angeordnet sein. Sie können im beliebigen Winkel zueinander versetzt an der Rührwelle 8 angeordnet sein.

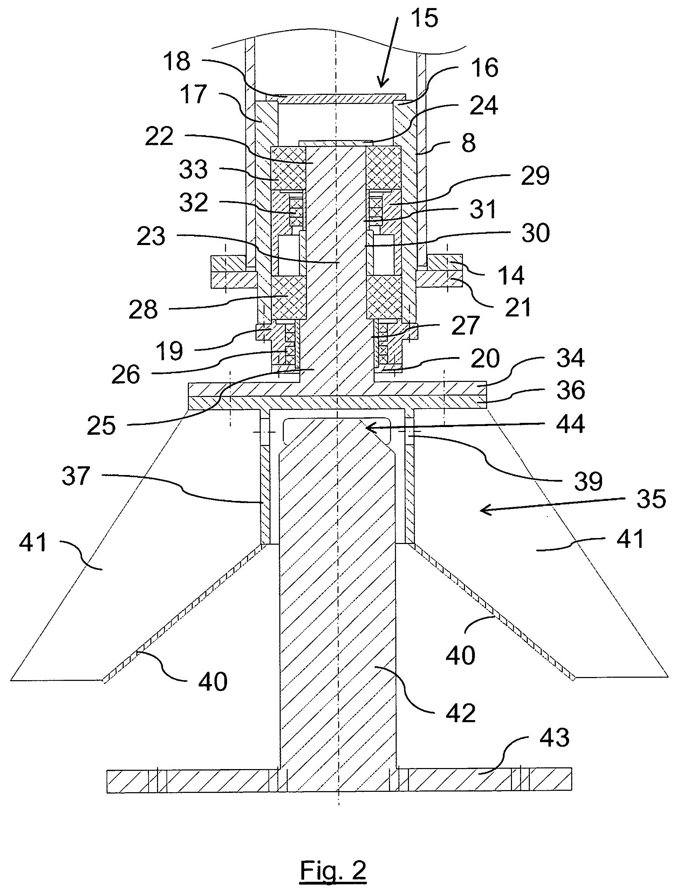

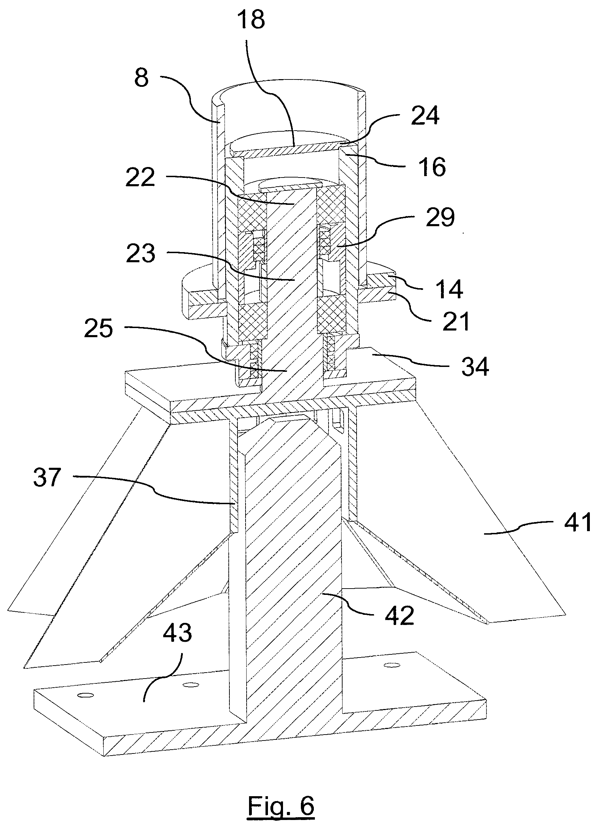

Die Rührpaddel 10 und die Paddelstangen 11 weisen jeweils Flansche mit korrespondierenden Bohrungen auf (nicht dargestellt), die mittels Schraubverbindungen miteinander befestigbar sind. Hierdurch ist es auch möglich, die Rührpaddel 10 gegenüber der Vertikalen mit unterschiedlichen Neigungswinkeln an den Paddelstangen 11 zu befestigen. Dies erlaubt eine Einstellung der effektiven Verdrängungsfläche der Rührpaddel 10. Je steiler die Rührpaddel 10 angeordnet sind, desto größer ist die effektive Verdrängungsfläche. Durch eine Schrägstellung der Rührpaddel 10 ist es auch möglich, eine vertikale Strömungskomponente im Substrat einzustellen. Die Figuren 2 und 6 zeigen den unteren Endbereich der Rührwelle 8 jeweils in einer Schnittdarstellung. Die Rührwelle 8 weist an ihrem unteren Rand einen radial nach außen vorstehenden Rührwellenflansch 14 auf. Im unteren Endbereich der Rührwelle 8 ist ein Lagereinsatz 15 eingesetzt. Der Lagereinsatz 15 weist ein mehrteiliges Gehäuse 16 auf. Das Gehäuse 16 umfasst einen etwa zylinderförmigen Gehäusegrundkörper 17, einen oberen, scheibenförmigen Deckel 18, einen unten am Gehäusegrundkörper 17 angeordneten Ringkörper 19 und eine an der Unterseite des Ringkörpers 19 angeordnete Ringplatte 20. Die einzelnen Gehäuseteile, der Gehäusegrundkörper 17, der Deckel 18, der Ringkörper 19 und die Ringplatte 20 sind mittels Schraubverbindungen miteinander lösbar verbunden. Am Außenumfang des Gehäusegrundköpers 17 ist ein radial nach außen vorstehender Lagerflansch 21 angeordnet, der korrespondierend bzw. kompatibel zum Rührwellenflansch 14 ausgebildet ist, so dass der Lagereinsatz 15 an der Rührwelle 8 durch Verschrauben der beiden Flansche 14, 21 lösbar befestigt ist. Im Lagereinsatz 15 ist ein Wellenstummel 22 angeordnet. Der Wellenstummel 22 weist einen Lagerabschnitt 23 auf, der einen geringeren Durchmesser als die übrigen Bereiche des Wellenstummels besitzt, so dass am oberen Ende des Wellenstummels 22 ein umlaufender Ringsteg 24 und am unteren Endbereich des Wellenstummels 22 ein Wellenabschnitt 25 mit größerem Durchmesser als der Lagerabschnitt ausgebildet sind.

Ein Teil des Wellenabschnittes 25 des Wellenstummels 22 wird vom Ringkörper19 und der Ringplatte 20 des Gehäuses 16 des Lagereinsatzes 15 umgeben. An der Innenseite des Ringkörpers 19 sind Dichtelemente 26 vorgesehen, die den Wellenabschnitt 25 gegenüber dem Gehäuse 16 des Lagereinsatzes 15 abdichten. Die Dichtelemente sind im vorliegenden Ausführungsbeispiel Simmerringe. Sie werden gegen ein Herausfallen nach unten durch die Ringplatte 20 gesichert. Zwischen den Dichtelementen 26 und dem Wellenstummel 22 ist eine Buchse 27 angeordnet. Auf dem oberen Rand der Buchse 27 und auf einem am Ringkörper 19 nach oben vorstehenden Vorsprung liegt ein unteres Lager 28 auf, das den Wellenstummel 22 drehbar gegenüber dem Gehäuse 16 des Lagereinsatzes 15 lagert. Im vorliegenden Ausführungsbeispiel ist das Lager als Pendelrollenlager ausgebildet. Es sind jedoch auch andere Arten von Kugel-, Walz- und Rollenlager möglich. Auf dem unteren Lager sind eine äußere Abstandsbuchse 29 und zwei innere Abstandsbuchsen 30, 31 angeordnet. Die untere innere Abstandsbuchse 30 begrenzt mit der äußeren Abstandsbuchse 29 einen Hohlraum oberhalb des unteren Lagers 28, der als Fettreservoir dient. Zwischen der oberen inneren Abstandsbuchse 31 und der äußeren Abstandsbuchse 29 sind weitere Dichtelemente 32 vorgesehen. Die Dichtelemente 32 sind wiederum als Simmerringe ausgebildet. Auf der äußeren Abstandsbuchse 29 und der oberen inneren Abstandsbuchse 31 liegt ein oberes Lager 33 auf. Das obere Lager ist wiederum als Pendelrollenlager ausgebildet. Die Buchsen 27, 31 schützen den Wellenstummel 22 vor den Simmerringen. Die Simmerringe würden ansonsten im Betrieb Rillen in die Buchse schleifen. Ohne die Buchsen 27, 31 würde der Wellenstummel 22 beschädigt werden und müsste beim Lagerwechsel entsorgt werden.

Bei einer nach unten gerichteten Belastung des Wellenstummels 22 stützt sich dieser mit seinem am oberen Rand ausgebildeten Ringsteg 24 am oberen Rand des oberen Lagers 33 nach unten hin ab. Bei einer nach oben gerichteten Belastung des Wellenstummels 22 stützt sich dieser mit seinem Wellenabschnitt 25 am unteren Rand des unteren Lagers 28 ab. Der Wellenstummel 22 ist somit drehbar, aber nicht axial verschieblich im Lagereinsatz 15 gelagert.

Der Deckel 18 des Gehäuses 16 des Lagereinsatzes 15 ist mit Abstand zum oberen Ende des Wellenstummels 22 angeordnet, so dass zwischen dem Wellenstummel und dem Deckel 18 ein Hohlraum ausgebildet ist. Dieser Hohlraum dient als Fettreservoir zum Schmieren des oberen Lagers 33. Am unteren Ende des Wellenstummels 22 ist ein rechteckiger, insbesondere quadratischer Flansch 34 befestigt. An diesem Flansch 34 ist ein Findungstrichter 35 befestigt. Der Findungstrichter 35 weist einen rechteckigen bzw. quadratischen Flansch 36 auf, der korrespondierend bzw. kompatibel zum Flansch 34 des Lagereinsatzes 15 ausgebildet ist, so dass der Findungstrichter 35 mittels Schraubverbindungen lösbar am Lagereinsatz befestigbar ist. Am Flansch 36 ist zentrisch ein nach unten vorstehender Zentrierabschnitt 37 ausgebildet. Der Zentrierabschnitt 37 besteht aus vier vertikal angeordneten Wandungen, die in der Draufsicht ein Quadrat begrenzen. Im Rahmen der Erfindung können auch eine unterschiedliche Anzahl von Wandungen vorgesehen sein, die vorzugsweise eine polygonale Form begrenzen. Am oberen Randbereich der Wandungen 38 des Zentrierabschnittes 37 sind Durchgangsöffnungen 39 eingebracht. Am unteren Endbereich des Zentrierabschnittes 37 schließt sich ein Trichterabschnitt 40 an, der nach unten aufweitend ausgebildet ist. Außenseitig sind am Zentrierabschnitt 37 und am Trichterabschnitt 40 vertikal stehende Stützwandungen 41 angebracht, die den Findungstrichter 35 versteifen.

Ein Zentrierstummel 42 steht senkrecht auf einem Bodenflansch 43. Der Zentrierstummel weist zumindest im oberen Endbereich eine Form auf, die der Form des Zentrierabschnittes 37 entspricht, so dass der Zentrierabschnitt 37 den Zentrierstummel 42 formschlüssig mit geringem Spiel umgreift. Die oberen Umfangskanten des Zentrierstummels 42 sind vorzugsweise als Einführschrägen 44 ausgebildet.

Der Bodenflansch 43 ist an der Bodenplatte 4 des Fermenters 1 mittels Schrauben befestigt. Der Zentrierstummel 42 steht von der Bodenplatte bzw. dem Bodenflansch senkrecht nach oben.

Die Rührwelle 8 eines Rührwerkes 7 kann einfach durch Absenken der Rührwelle im Substrat des Fermenters eingesetzt bzw. durch Anheben und Herausziehen aus dem Fermenter ausgetauscht werden. Beim Einführen eines Rührwerkes in einen mit Substrat gefüllten Fermenter strömt das Substrat durch den Trichterabschnitt 40 und den Zentrierabschnitt 37 und tritt an den Durchgangsöffnungen 39 seitlich aus. Hierdurch wird der vom Findungstrichter 35 verursachte Strömungswiderstand erheblich herabgesetzt. Der Findungstrichter 35 bremst das Einführen der Rührwelle in einen Fermenter, jedoch ist durch das Vorsehen der Durchgangsöffnungen 39 sichergestellt, dass die Rührwelle aufgrund ihres Eigengewichtes, selbst in einem sehr viskosen Substrat, vertikal eintaucht und nicht seitlich ausbricht. Die Größe und die Anzahl der Durchgangslöcher können entsprechend dem Gewicht des Rührwerkes, der Viskosität des Substrates und der Verdrängungsfläche des Findungstrichters angepasst werden. Es können auch weitere Durchgangsöffnungen im Bereich des Trichterabschnittes angeordnet sein.

Je größer der Findungstrichter ist, desto einfacher kann die Kopplung mit dem Zentrierstummel 42 erzielt werden. Deshalb ist es zweckmäßig, die Trichterfläche, d.h. die vom Randbereich des Trichterabschnitts 40 umfasste Fläche zumindest 900 cm² groß, insbesondere 1.000 cm² und vorzugsweise mindestens 1.500 cm² auszubilden. Je größer die vom Trichterabschnitt 40 umfasse Trichterfläche ist, desto größer ist der Strömungswiderstand im Substrat, weshalb diese Trichterfläche nicht größer als 10.000 cm², insbesondere nicht größer als 7.500 cm² und vor allem nicht größer als 5.000 cm² sein soll.

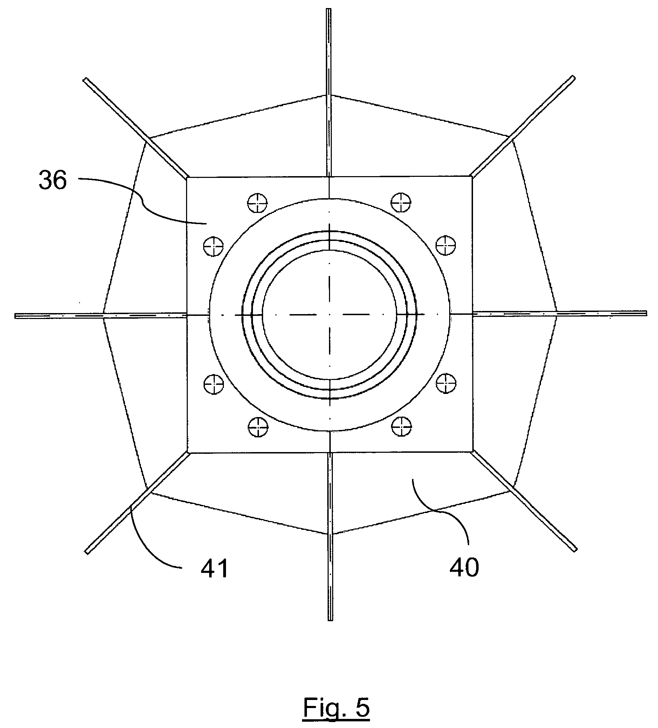

Figur 5 zeigt den Findungstrichter 35 in einer Ansicht von oben, wobei man erkennen kann, dass der Trichterabschnitt 40 8-eckig ausgebildet ist.

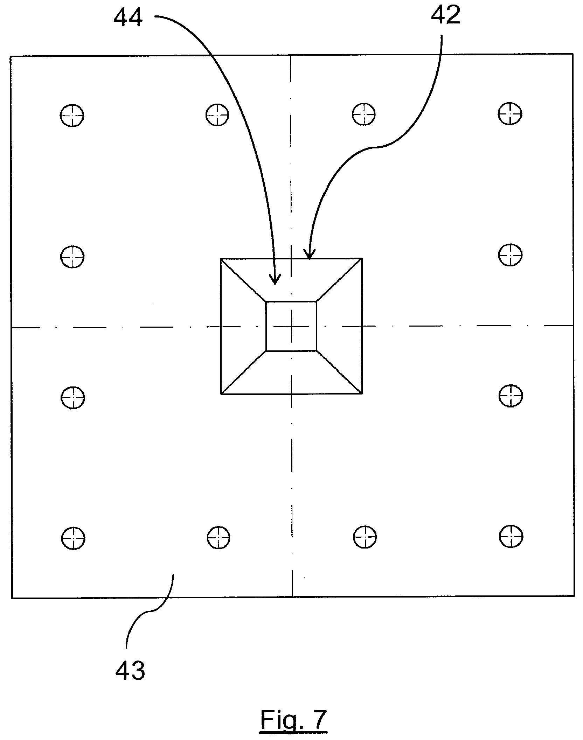

Figur 7 zeigt den Zentrierstummel 42 und den Bodenflansch 43 in einer Draufsicht. Der Bodenflansch 43 weist mehrere Bohrungen auf, an welchen der Bodenflansch mittels Schrauben an der Bodenplatte befestigt werden kann.

**Bezugszeichenliste**

| | | | |
|---|---|---|---|
| 1 | Fermenter | 24 | Ringsteg |
| 2 | Fermenterkörper | 25 | Wellenabschnitt |
| 3 | | 26 | Dichtelement |
| 4 | Bodenplatte | 27 | Buchse |
| 5 | Seitenwand | 28 | unteres Lager |
| 6 | Decke | 29 | äußere Abstandsbuchse |
| 7 | Rührwerk | 30 | innere Abstandsbuchse |
| 8 | Rührwelle | 31 | innere Abstandsbuchse |
| 9 | Antriebsmechanismus | 32 | Dichtelement |
| 10 | Rührpaddel | 33 | oberes Lager |
| 11 | Paddelstange | 34 | Flansch |
| 12 | Deckelplatte | 35 | Findungstrichter |
| 13 | Öffnung | 36 | Flansch |
| 14 | Rührwellenflansch | 37 | Zentrierabschnitt |
| 15 | Lagereinsatz | 38 | Wandung |
| 16 | Gehäuse | 39 | Durchgangsöffnung |
| 17 | Gehäusegrundkörper | 40 | Trichterabschnitt |
| 18 | Deckel | 41 | Stützwandung |
| 19 | Ringkörper | 42 | Zentrierstummel |
| 20 | Ringplatte | 43 | Bodenflansch |
| 21 | Lagerflansch | 44 | Einführschräge |
| 22 | Wellenstummel | | |
| 23 | Lagerabschnitt | | |

## Patentansprüche

1. Rührwerk für einen Fermenter mit
- einer etwa vertikal angeordneten Rührwelle (8), an welcher zumindest ein Paddel (10) angeordnet ist,
- einem Antriebsmechanismus (9) zum Drehen der Rührwelle (8), wobei der Antriebsmechanismus (9) am oberen Endbereich der Rührwelle (8) angreift,
- einem Findungstrichter (35), der am unteren Ende der Rührwelle (8) angeordnet ist, wobei der Findungstrichter (35) einen Zentrierabschnitt (37) und einen nach unten sich aufweitenden Trichterabschnitt (40) aufweist,
- einem an einem Boden eines Fermenters befestigbaren Zentrierstummel (42) zum formschlüssigen Eingreifen in den Zentrierabschnitt (37),
- einen Lagereinsatz (15), der am unteren Ende der Rührwelle (8) angeordnet ist und einen mit dem Findungstrichter (35) verbundenen Wellenstummel (22) drehbar gegenüber der Rührwelle (8) lagert,
**dadurch gekennzeichnet, dass**
der Trichterabschnitt (40) und/oder der Zentrierabschnitt (37) zumindest eine Öffnung (39) aufweist, so dass beim Eintauchen des Findungstrichters (35) in ein in einem Fermenter enthaltenes Substrat dieses durch den Findungstrichter (35) hindurch strömen kann.

2. Rührwerk für einen Fermenter nach Anspruch 1 mit
- einer etwa vertikal angeordneten Rührwelle (8), an welcher zumindest ein Paddel (10) angeordnet ist,
- einem Antriebsmechanismus (9) zum Drehen der Rührwelle (8), wobei der Antriebsmechanismus (9) am oberen Endbereich der Rührwelle (8) angreift,
- einem Findungstrichter (35), der einen Zentrierabschnitt (37) und einen Trichterabschnitt (40) aufweist,
- einen Lagereinsatz (15), der am unteren Ende der Rührwelle (8) angeordnet ist und einen mit dem Findungstrichter verbundenen bzw. koppelbaren Wellenstummel drehbar gegenüber der Rührwelle lagert,
**dadurch gekennzeichnet, dass** der Lagereinsatz lösbar mit der Rührwelle verbunden ist.

3. Rührwerk nach Anspruch 1 oder 2,
**dadurch gekennzeichnet,**
**dass** der Findungstrichter (35) lösbar mit dem Wellenstummel (22) verbunden ist.

4. Rührwerk nach einem der Ansprüche 1 bis 3,
**dadurch gekennzeichnet,**
**dass** die lösbare Verbindung zwischen dem Lagereinsatz (15) und der Rührwelle (8) und/oder zwischen dem Findungstrichter (35) und dem Wellenstummel (22) jeweils aus zwei kompatiblen Flanschen (14, 21; 34, 44) ausgebildet ist, welche miteinander verschraubbar sind.

5. Rührwerk nach einem der Ansprüche 1 bis 4,
**dadurch gekennzeichnet,**
**dass** der Zentrierabschnitt (37) in der Draufsicht die Form eines Polyeders, insbesondere eines Quadrates, besitzt.

6. Rührwerk nach einem der Ansprüche Anspruch 1 bis 5,
**dadurch gekennzeichnet,**
**dass** die Rührwelle (8) einen rohrförmigen Wellenkörper umfasst, in dem der Lagereinsatz (15) eingesetzt ist.

7. Rührwerk nach einem der Ansprüche 1 bis 6,
**dadurch gekennzeichnet,**
**dass** der Lagereinsatz (15) zumindest ein oder mehrere Kugel-, Wälz-, Rollen-, Pendelrollen- und/oder Nadellager (28, 33) aufweist.

8. Rührwerk nach einem der Ansprüche 1 bis 7,
**dadurch gekennzeichnet,**
**dass** der Lagereinsatz (15) zumindest zwei Lager (28, 33) aufweist, welche zwischen dem Wellenstummel (22) und einem Lagereinsatzgehäuse (16) angeordnet und voneinander beabstandet sind.

9. Rührwerk nach Anspruch 7 oder 8,
**dadurch gekennzeichnet,**
**dass** benachbart zum Lager (28, 33) an der zum Findungstrichter (35) weisenden Seite ein Dichtelement (26, 32) zwischen dem Wellenstummel (22) und dem Lagersatzgehäuse (16) angeordnet ist.

10. Rührwerk nach einem der Ansprüche 1 bis 9,
**dadurch gekennzeichnet,**
**dass** benachbart zum Lager (28, 33) an der vom Findungstrichter (35) weg weisenden Seite ein als Fettreservoir dienender Hohlraum vorgesehen ist.

11. Fermenter, mit
- einem Gehäuse (2) umfassend zumindest eine Bodenplatte (4) und eine oder mehrere die Bodenblatte (4) umschließenden Seitenwandungen (5),
- einem Rührwerk (7), das nach einem der Ansprüche 1 bis 10 ausgebildet ist,
- einer Eintragseinrichtung zum Zuführen von Inputstoffen, und
- eine Entnahmeöffnung.

12. Fermenter nach Anspruch 11,
**dadurch gekennzeichnet,**
**dass** der Fermenter zumindest zwei Rührwerke (7) aufweist und/oder, dass das oder die Rührwerke (7) eine vertikal angeordnete Rührwelle (8) aufweisen.

13. Fermenter nachAnspruch 11 oder 12,
**dadurch gekennzeichnet,**
**dass** die Eintragseinrichtung zum Zuführen der Inputstoffe am oberen Bereich des Fermenters (1) und die Entnahmeöffnung am unteren Bereich des Fermenters (1) angeordnet sind, wobei
die Entnahmeöffnung vorzugsweise am Fermenter (1) diametral gegenüberliegend zur Eintragseinrichtung angeordnet ist.

14. Fermenter nach einem der Ansprüche 11 bis 13,
**dadurch gekennzeichnet,**
**dass** die Seitenwandung (5) des Fermenters in der Draufsicht kreisförmig ausgebildet ist, und/oder der Fermenter (1) eine Decke (6) aufweist, und/oder wobei die Seitenwandung (5) und ggfs. die Decke (6) aus Beton-Fertigteilen ausgebildet sind.

15. Verfahren zum Erzeugen von Biogas mit einem Fermenter nach einem der Ansprüche 11 bis 14, wobei an der Eintragseinrichtung Inputstoffe zugeführt werden, im Gehäuse unter laminarer Umwälzung fermentiert und an der Entnahmeöffnung abgezogen werden.

## Claims

1. Agitator for a fermenter with
- an approximately vertically arranged agitator shaft (8), on which at least one paddle (10) is disposed,
- a drive mechanism (9) for rotating the agitator shaft (8), wherein the drive mechanism (9) engages at the upper end area of the agitator shaft (8),
- a finding funnel (35), which is arranged at the lower end of the agitator shaft (8), wherein the finding funnel (35) comprises a centering section (37) and a funnel section (40) widening downwards,
- a centering nub (42) attachable to a base of a fermenter for positive-locking engagement in the centering section (37),
- a bearing insert (15), which is arranged at the lower end of the agitator shaft (8) and which bears a shaft end (22) connected with the finding funnel (35) pivotally with respect to the agitator shaft (8),
**characterized in that**
the funnel section (40) and/or the centering section (37) comprise(s) at least one opening (39), so that upon immersion of the finding funnel (35) into a substrate comprised in a fermenter the substrate may flow throughout the finding funnel (35).

2. Agitator for a fermenter according to claim 1 with
- an approximately vertically arranged agitator shaft (8) on which at least one paddle (10) is disposed,
- a drive mechanism (9) for rotating the agitator shaft (8), wherein the driving mechanism (9) engages at the upper end section of the driving shaft (8),
- a finding funnel (35) which comprises a centering section (37) and a funnel section (40),
- a bearing insert (15), which is arranged at the lower end of the agitator shaft (8) and which pivotally bears with respect to the agitator shaft a shaft end connected or connectable to the finding funnel,
**characterized in that**
the bearing insert is detachably connected to the agitator shaft.

3. Agitator according to claim 1 or 2,
**characterized in that**
the finding funnel (35) is connected to the shaft end (22) in a detachable way.

4. Agitator according to one of the claims 1 to 3,
**characterized in that**
the detachable connection between the bearing insert (15) and the agitator shaft (8) and/or between the finding funnel (35) and the shaft end (22) is respectively formed by two compatible flanges (14, 21; 34, 44), which can be screwed together.

5. Agitator according to one of the claims 1 to 4,
**characterized in that**
the centering section (37) in the plan view possesses the shape of a polyhedron, in particular of a square.

6. Agitator according to one of the claims 1 to 5,
**characterized in that**
the agitator shaft (8) comprises a tubular shaft body, wherein the bearing insert (15) is inserted.

7. Agitator according to one of the claims 1 to 6,
**characterized in that**
the bearing insert (15) comprises at least one or several ball, roller, rolling, spherical roller and/or needle bearings (28, 33).

8. Agitator according to one of the claims 1 to 7,
**characterized in that**
the bearing insert (15) comprises at least two bearings (28, 33), which are arranged between the shaft end (22) and a bearing insert housing (16) and spaced from one another.

9. Agitator according to claim 7 or 8,
**characterized in**
**that** adjacent to the bearing (28, 33) at the side pointing towards the finding funnel (35) a sealing element (26, 32) is disposed between the shaft end (22) and the bearing insert housing (16).

10. Agitator according to one of the claims 1 to 9,
**characterized in that**
adjacent to the bearing (28, 33) at the side pointing away from the finding funnel (35) a cavity serving as a lubricant reservoir is provided.

11. Fermenter with
- a housing (2) comprising at least one base plate (4) and one or more side walls (5) enclosing the base plate (4),
- an agitator (7), which is formed according to one of the claims 1 to 10,
- an input equipment for the feeding of input materials, and
- a removal opening.

12. Fermenter according to claim 11,
**characterized in that**
the fermenter comprises at least two agitators (7) and/or, that the agitator(s) (7) comprise(s) a vertically arranged agitator shaft (8).

13. Fermenter according to claim 11 or 12,
**characterized in that**
the input equipment for the feeding of the input materials is situated at the upper end of the fermenter (1) and that the removal opening is situated at the lower end of the fermenter (1), wherein
the removal opening is preferably arranged on the fermenter (1) diametrically opposite the entering equipment.

14. Fermenter according to one of the claims 11 to 13,
**characterized in that**
the side wall (5) of the fermenter in plan view is formed circularly, and/or the fermenter (1) comprises a ceiling (6) and/or wherein the side wall (5) and optionally the ceiling (6) is formed of pre-cast concrete elements.

15. Process for generating biogas in a fermenter according to one of claims 11 to 14, wherein input materials are fed at the entering equipment, fermented within the housing under laminar circulation and removed at the removal opening.

## Revendications

1. Agitateur pour appareil de fermentation avec
- un arbre d'agitation disposé à peu près verticalement (8), sur lequel au moins une pagaie (10) est arrangée,
- un mécanisme d'entrainement (9) pour tourner l'arbre agitateur (8), dans lequel le mécanisme d'entrainement (9) vient en contact dans la partie d'extrémité supérieure de l'arbre agitateur (8),
- un entonnoir de repérage (35) qui se situe à l'extrémité inférieure de l'arbre agitateur, dans lequel l'entonnoir de repérage(35) comprend une section de centrage (37) et une section d'entonnoir s'élargissant vers le bas (40),
- un embout de centrage (42) pouvant être fixé sur le sol de l'appareil de fermentation pour venir en prise par complémentarité de forme avec la section de centrage (37),
- un insert de palier (15), qui se situe à l'extrémité inférieure de l'arbre agitateur (8) et qui porte un embout d'arbre (22) connecté à l'entonnoir de repérage (35) pivotant par rapport à l'arbre agitateur (8),
- **caractérisé en ce**
**que** la section d'entonnoir (40) et/ou la section de centrage (37) comprend au moins une ouverture (39), de manière à ce que, lors de l'immersion de l'entonnoir de repérage (35) dans un substrat contenu dans un appareil de fermentation, celui-ci peut circuler à travers l'entonnoir de repérage (35).

2. Agitateur pour un appareil de fermentation selon la revendication 1, avec
- un arbre agitateur (8) disposé à peu près verticalement, auquel au moins une pagaie (10) est appliquée,
- un mécanisme d'entrainement (9) pour tourner l'arbre agitateur (8), dans lequel le mécanisme d'entrainement (9) intervient à l'extrémité supérieure de l'arbre agitateur (8),
- un entonnoir de repérage (35), qui comprend une section de centrage (37) et une section d'entonnoir (40),
- un insert de palier (15), situé à l'extrémité inférieure de l'arbre agitateur (8) et portant un embout d'arbre connecté ou connectable à l'entonnoir de repérage pivotant par rapport à l'arbre agitateur,
**caractérisé en ce**
**que** l'insert de palier est connecté à l'arbre agitateur de manière amovible.

3. Agitateur selon la revendication 1 ou 2,
**caractérisé en ce**
**que** l'entonnoir de repérage (35) est connecté de manière amovible à l'embout d'arbre (22).

4. Agitateur selon une des revendications 1 à 3,
**caractérisé en ce**
**que** la connexion amovible entre l'insert de palier (15) et l'arbre agitateur (8) et/ou entre l'entonnoir de repérage (35) et l'embout d'arbre (22) est respectivement formé par deux brides compatibles (14, 21; 34, 44), lesquelles peuvent être vissées les unes avec les autres.

5. Agitateur selon une des revendications 1 à 4,
**caractérisé en ce**
**que** la section de centrage (37) en vue de dessus possède la forme d'un polyèdre, plus particulièrement d'un carré.

6. Agitateur selon une des revendications 1 à 5,
**caractérisé en ce**
**que** l'arbre agitateur (8) comprend un corps d'arbre tubulaire, dans lequel l'insert de palier (15) est inséré.

7. Agitateur selon une des revendications 1 à 6,
**caractérisé en ce**
**que** l'insert de palier (15) comprend au moins un ou plusieurs roulement(s) à billes, mécaniques à rouleaux, à rotule et/ou à aiguille (28, 33).

8. Agitateur selon une des revendications 1 à 7,
**caractérisé en ce**
**que** l'insert de palier (15) comprend au moins deux paliers (28, 33) se situant entre l'embout d'arbre (22) et un boitier d'insert de palier (16) et qui sont écartés l'un del'autre.

9. Agitateur selon la revendication 7 ou 8,
**caractérisé en ce**
**qu'**adjacent au palier (28, 33) sur le côté montrant vers l'entonnoir de repérage (35) un élément d'étanchéité (26, 32) est disposé entre l'embout d'arbre (22) et le boitier d'insert de palier (16).

10. Agitateur selon une des revendications 1 à 9,
**caracterisé en ce**
**qu'**adjacent au palier (28, 33) sur le côté s'écartant de l'entonnoir de repérage (35) une cavité servant en tant que réservoir de lubrifiant est prévue.

11. Appareil de fermentation avec
- un boitier (2) comprenant au moins une plaque de base (4) et une ou plusieurs paroi(s) latérale(s) (5) enfermant la plaque de base (4),
- un agitateur (7), qui est formé selon une des revendications 1 à 10,
- un dispositif pour introduire des matériaux entrant, et
- une ouverture de prélèvement.

12. Appareil de fermentation selon la revendication 11,
**caractérisé en ce**
**que** l'appareil de fermentation comprend au moins deux agitateurs (7) et/ou, que le ou les agitateur(s) (7) comprennent un arbre agitateur (8) disposé verticalement.

13. Appareil de fermentation selon la revendication 11 ou 12,
**caractérisé en ce**
**que** le dispositif pour l'introduction des matériaux entrant est situé à la zone supérieure de l'appareil de fermentation (1) et l'ouverture de prélèvement est située à la zone inférieure de l'appareil de fermentation (1),
l'ouverture de prélèvement étant préférablement située à l'appareil de fermentation (1) diamétralement opposé au dispositif d'introduction.

14. Appareil de fermentation selon une des revendications 11 à 13,
**caractérisé en ce**
**que** la paroi latérale (5) de l'appareil de fermentation en vue de dessus est formée de manière circulaire et/ou que l'appareil de fermentation (1) comprend un plafond (6), et/ou dans lequel la paroi latérale (5) et facultativement le plafond (6) est effectué en préfabriqués de béton.

15. Procédé pour la génération de biogaz dans un appareil de fermentation selon une des revendication 11 à 14, dans lequel des matériaux d'entrée sont introduits au dispositif d'introduction, fermenté dans le boitier sous une circulation laminaire, et retirés à l'ouverture de prélèvement.
